# EUROPEAN PATENT SPECIFICATION

(11) **EP 1 964 832 B1**
(45) Date of publication and mention of the grant of the patent: **23.05.2012**
(21) Application number: 07004301.3
(22) Date of filing: 02.03.2007
(51) Int. Cl.: C07C 219/06, C07C 219/08, A61Q 19/10, A61Q 5/12, A61K 8/44, C11D 1/62

(54) **Polymeric esterquats with asymmetric side chains**
Polymeresterquats mit asymmetrischen Seitenketten
Esterquats polymères à chaînes latérales asymétriques

(43) Date of publication of application: 03.09.2008
(73) Proprietor: Cognis IP Management GmbH, 40589 Düsseldorf (DE)
(72) Inventor: Bigorra Llosas, Joaquin, Dr., 08201 Sabadell (ES); Behler, Ansgar, Dr., 46240 Bottrop (DE); Amela Conesa, Cristina, 08290 Cerdanyola del Valles (ES)
(74) Representative: Fabry, Bernd

(56) References cited:
- EP-A- 1 160 238
- US-A- 5 880 299
- US-A1- 2004 180 028
- US-B1- 6 300 307

## Description

### Field of the invention

The present invention relates to the area of cosmetics and refers to new polymeric cationic surfactants with asymmetric side chains, especially useful for applications in hair and skin care.

### Background of the invention

"Esterquats" are generally understood to be quaternised fatty acid triethanolamine ester salts which are broadly suitable both for softening fibres and for conditioning hair. In the last decade, these substances have significantly displaced conventional quaternary ammonium compounds from the market, for example, distearyl dimethyl ammonium chloride, by virtue of their better eco-toxicological compatibility. Reviews of this subject have been published since the early 1990s, for example, by O. Ponsati in C. R. CED-Congress, Barcelona, 1992, page 167**,** by R. Puchta et al. in Tens. Surf. Det. 30 , 186 (1993**),** by M. Brock in Tens. Surf. Det. 30, 394 (1993**)** and by R. Lagerman et al. in J. Am. Oil. Chem. Soc., 71, 97 (1994**).** Since that time various types of esterquats and also "amidequats" have been synthesized so that a huge state of the art is now available for everyone looking for an adequate esterquat to solve his specific problem. Nevertheless, one can still find gaps where the products known from the market or even from literature do not meet the requirements of the market, especially in cases where the customer expects that the products fit into a complex profile.

For example, EP0770594B1(Henkel) discloses polymeric esterquats obtainable by esterification of blends of mono- an dicarboxylic acids with alkanolamines and subsequent quaternisation of the mixtures of mono-, di- and triesters by means of suitable alkylation agents. The polymeric structure is achieved by using about 50 % of dicarboxylic acids bridging the monomeric structures which stem from the monocarboxylic, more particularly fatty acids. The document teaches that fatty acids are suitable with respect to the monocarboxylic acid part of the molecule C6 to C22, while the use of medium/long chain fatty acids of 12 to 18 carbon atoms represent the preferred embodiment of the invention. The preferred dicarboxylic acid is adipic acid, while the molar ratio of the two components forming the backbone of the molecule, so to speak, is variable between 1:10 and 10:1. As a matter of fact, the esterquats disclose only those species having long or medium symmetric side chains. Nevertheless, the major advantage compared to the state of the art stems from the fact that the products can act both as a cationic surfactant or a cationic polymer in a formulation. The disadvantage however is that the surfactant properties and especially the emulsifying properties are dominated by the polymeric behaviour of the molecules. It has therefore been the object of the present invention to modify the structure of said polymeric esterquats in order to better balance surfactant and polymer qualities of the products and to improve their all-over performance in cosmetic compositions, particularly in hair care products.

US2004180028 discloses esterquats obtainable from a mixture of caprylic, capric and adipic acid which is reacted with triethanolamine and then (CH3)2SO4.

### Detailed description of the invention

The present invention refers to polymeric esterquats with asymmetric side chains, obtainable by reacting alkanolamines with a mixture of
(i) C₆-C₁₀ monocarboxylic acids
(ii) C₁₂-C₂₂ monocarboxylic acids, and
(iii) Dicarboxylic acids, and
quaternising the resulting esters with alkylation agents, optionally after alkoxylation.

Surprisingly it has been observed that balancing the chain length of the monocarboxylic acid part of the molecules improves the surfactant properties of the products without influencing the polymeric properties negatively. More particularly, it has been found that replacing the medium/long chain fatty acids by a mixture of short chain and medium/long chain fatty acids, which changes the structure of the molecule by incorporation of asymmetric side chains, solves the problem underlying the present invention.

### Manufacturing process

The polymeric esterquats with asymmetric side chains according to the present invention represent new cationic surfactants. More particularly, the polymeric esterquats are obtained by reacting alkanol amines with a mixture of short chain fatty acids, medium/long chain fatty acids and dicarboxylic acids, and quaternising the resulting esters with alkylation agents in known manner, optionally after alkoxylation.

According to the present invention, suitable polymeric esterquats are derived from alkanolamines derived from amines following general formula **(I).** 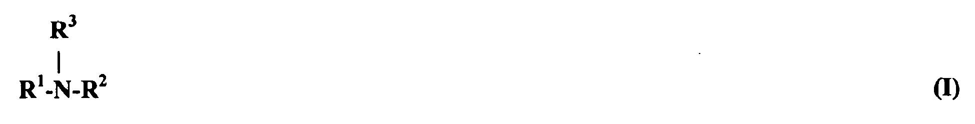 in which R¹ represents a hydroxyethyl radical, and R² and R³ independently of one another stand for hydrogen, methyl or a hydroxyethyl radical. Typical examples are methyldiethanolamin (MDA), monoethanolamine (MES), diethanolamine (DEA) and triethanolamine (TEA). In a preferred embodiment of the present invention, triethanolamine is used as the starting material.

On the other hand, in a further preferred embodiment of the present invention, it is possible to use mixtures of
(i) Monocarboxylic acids selected from the group consisting of caproic acid, caprylic acid, 2-ethyl hexanoic acid, caprinic acid and their mixtures,
(ii) Monocarboxylic acids selected from the group consisting of lauric acid, myristic acid, palmitic acid, stearic acid, oleic acid, behenic acid, erucic acid and their mixtures, and
(iii) Dicarboxylic acids according to general formula (II),

   **HOOC-[X]-COOH** **(II)**

   in which [X] stands for an optionally hydroxysubstituted alk(en)ylene group having 1 to 10 carbon atoms.

It has to be understood that the fatty acids representing group (i) and (ii) may also encompass technical grade fatty acids mixtures which can be derived from the splitting of fats and oils, optionally after additional separation and distillation, and therefore may also include other species.

Dicarboxylic acids (iii) suitable for use as starting materials in accordance with the invention are typically selected from the group consisting of succinic acid, maleic acid, glutaric acid, 1,12-dodecanedioic acid. The best results, however, are obtained by incorporating adipic acid into the polymeric esterquat. The over-all preferred polymeric esterquats are obtained from mixtures consisting of caprylic acid, stearic acid and adipic acid.

With respect to the properties of the surfactants in the final hair or skin care products it has been found rather advantageous to use the monocarboxylic acids forming the groups (i) and (ii) in molar ratios of about 30:70 to about 70:30, and preferably in a ratio of about 50:50.

The fatty acids (i+ii) and the dicarboxylic acids (iii) may be used in a molar ratio of 1:10 to 10:1. However, it has proved to be of advantage to adjust a molar ratio of 1:1 to 2:1. The trialkanolamines on the one hand and the acids - i.e. fatty acids and dicarboxylic acids together - on the other hand may be used in a molar ratio of 1:1 to 1:2. A molar ratio of trialkanolamine to acids of 1:1.2 to 1:1.5 has proved to be optimal. The esterification may be carried out in known manner, for example as described in International patent application WO 91/01295 (Henkel). In one advantageous embodiment, it is carried out at temperatures between 120 °C and 220 °C, and more particularly between 130 °C and 170 °C under pressures of 0.01 to 1 bar. Suitable catalysts are hypophosphorous acids and alkali metal salts thereof, preferably sodium hypophosphite, which may be used in quantities of 0.01 to 0.1% by weight, and preferably in quantities of about 0.05 to about 0.07 % b.w. based on the starting materials. In the interests of particularly high colour quality and stability, it has proved to be of advantage to use as co-catalysts alkali metal and/or alkaline earth metal borohydrides, for example, potassium, magnesium and, in particular, sodium borohydride. The co-catalysts are normally used in quantities of about 50 to about 1.000 ppm, and more particularly in quantities of about 100 to about 500 ppm, again based on the starting materials. Corresponding processes are also the subject of DE 4308792 C1 and DE 4409322 C1 (Henkel) to which reference is hereby specifically made. Alternatively, the esterification may be carried out with the two components in successive steps.

Polymeric esterquats containing polyalkylene oxide may be produced by two methods. Firstly, ethoxylated trialkanolamines may be used. This has the advantage that the distribution of alkylene oxide in the resulting esterquat is substantially the same in regard to the three OH groups of the amine. However, it also has the disadvantage that the esterification reaction is more difficult to carry out on steric grounds. Accordingly, the preferred method is to alkoxylate the ester before quaternisation. This may be done in known manner, i.e. in the presence of basic catalysts and at elevated temperatures. Suitable catalysts are, for example, alkali metal and alkaline earth metal hydroxides and alcoholates, preferably sodium hydroxide, and more preferably sodium methanolate. The catalysts are normally used in quantities of 0.5 to 5% by weight, and preferably in quantities of 1 to 3% by weight, based on the starting materials. Where these catalysts are used, free hydroxyl groups are primarily alkoxylated. However, if calcined hydrotalcites or hydrotalcites hydrophobicized with fatty acids are used as catalysts, the alkylene oxides are also inserted into the ester bonds. This method is preferred where the required alkylene oxide distribution approaches the one obtained in which alkoxylated trialkanolamines are used. Ethylene and propylene oxide and mixtures thereof (random or block distribution) may be used as alkylene oxides. The reaction is normally carried out at temperatures in the range from 100 °C to 180 °C. The incorporation of, on average, 1 to 10 moles of alkylene oxide per mole of ester increases the hydrophilicity of the esterquat, improves solubility and reduces reactivity to anionic surfactants.

The quaternisation of the fatty acid/dicarboxylic acid trialkanolamine esters may be carried out in known manner. Although the reaction with the alkylation agents may also be carried out in the absence of solvents, it is advisable to use at least small quantities of water or lower alcohols, preferably isopropyl alcohol, for the production of concentrates which have a solids content of at least 80% by weight, and more particularly at least 90% by weight. Suitable alkylation agents are alkyl or aryl halides such as, for example, methyl chloride, benzyl chloride dialkyl sulphates, such as dimethyl sulphate or diethyl sulphate, for example, or dialkyl carbonates, such as dimethyl carbonate or diethyl carbonate, for example. The esters and the alkylating agents are normally used in amounts of 95 to 105 Mol-% calculated on the molar amount of nitrogen within the ester mixture, i.e. in a substantially stoichiometric ratio. The reaction temperature is usually in the range from 40 °C to 80 °C, and more particularly in the range from 50 °C to 60 °C. After the reaction it is advisable to destroy unreacted alkylation agent by addition of, for example, ammonia, an (alkanol)amine, an amino acid or an oligopeptide as described, for example, in DE 14026184 A1 (Henkel).

### Industrial application

Another object of the present invention concerns the use of the polymeric esterquats with asymmetric side chains for making cosmetic compositions such as, for example, skin care or hair care composition, in particular for making shampoos or conditioners in which they can be present in amounts of 1 to 20, preferably 2 to 15, and more preferably 5 to 10 % b.w., calculated on the final composition.

### Cosmetic compositions

Compositions comprising the new polymeric esterquats with asymmetric side chains may contain co-surfactants, oil bodies, emulsifiers, superfatting agents, pearlising waxes, consistency factors, polymers, silicone compounds, waxes, stabilizers, primary and secondary sun protection agents, antidandruff agents, biogenic agents, film formers, swelling agents, hydrotropes, preservatives, solubilizers, complexing agents, reducing agents, alkalising agents, perfume oils, dyes and the like such as additional auxiliaries and additives.

### Co-surfactants

Other preferred auxiliaries and additives are anionic and/or amphoteric or zwitterionic surfactants. Typical examples of anionic surfactants are soaps, alkyl benzenesulphonates, alkanesulphonates, olefin sulphonates, alkylether sulphonates, glycerol ether sulphonates, methyl ester sulphonates, sulphofatty acids, alkyl sulphates, fatty alcohol ether sulphates, glycerol ether sulphates, fatty acid ether sulphates, hydroxy mixed ether sulphates, monoglyceride (ether) sulphates, fatty acid amide (ether) sulphates, mono- and dialkyl sulphosuccinates, mono- and dialkyl sulphosuccinamates, sulphotriglycerides, amide soaps, ether carboxylic acids and salts thereof, fatty acid isethionates, fatty acid sarcosinates, fatty acid taurides, N-acylamino acids such as, for example, acyl lactylates, acyl tartrates, acyl glutamates and acyl aspartates, alkyl oligoglucoside sulphates, protein fatty acid condensates (particularly wheat-based vegetable products) and alkyl (ether) phosphates. If the anionic surfactants contain polyglycol ether chains, they may have a conventional homolog distribution although they preferably have a narrow-range homolog distribution. Typical examples of amphoteric or zwitterionic surfactants are alkylbetaines, alkylamidobetaines, aminopropionates, aminoglycinates, imidazolinium betaines and sulphobetaines. The surfactants mentioned are all known compounds. Information on their structure and production can be found in relevant synoptic works, cf. for example J. Falbe (ed.), "Surfactants in Consumer Products", Springer Verlag, Berlin, 1987, pages 54 to 124 **or** J. Falbe (ed.), "Katalysatoren, Tenside und Mineralöladditive (Catalysts, Surfactants and Mineral Oil Additives)", Thieme Verlag, Stuttgart, 1978, pages 123-217**.** The percentage content of surfactants in the preparations may be from 0.1 to 10% by weight and is preferably from 0.5 to 5% by weight, based on the preparation.

### Oil bodies

Suitable oil bodies which form constituents of the O/W emulsions are, for example, Guerbet alcohols based on fatty alcohols having 6 to 18 carbon atoms, preferably 8 to 10 carbon atoms, esters of linear C₆-C₂₂-fatty acids with linear or branched C₆-C₂₂-fatty alcohols or esters of branched C₆-C₁₃-carboxylic acids with linear or branched C₆-C₂₂-fatty alcohols such as, for example, myristyl myristate, myristyl palmitate, myristyl stearate, myristyl isostearate, myristyl oleate, myristyl behenate, myristyl erucate, cetyl myristate, cetyl palmitate, cetyl stearate, cetyl isostearate, cetyl oleate, cetyl behenate, cetyl erucate, stearyl myristate, stearyl palmitate, stearyl stearate, stearyl isostearate, stearyl oleate, stearyl behenate, stearyl erucate, isostearyl myristate, isostearyl palmitate, isostearyl stearate, isostearyl isostearate, isostearyl oleate, isostearyl behenate, isostearyl oleate, oleyl myristate, oleyl palmitate, oleyl stearate, oleyl isostearate, oleyl oleate, oleyl behenate, oleyl erucate, behenyl myristate, behenyl palmitate, behenyl stearate, behenyl isostearate, behenyl oleate, behenyl behenate, behenyl erucate, erucyl myristate, erucyl palmitate, erucyl stearate, erucyl isostearate, erucyl oleate, erucyl behenate and erucyl erucate. Also suitable are esters of linear C₆-C₂₂-fatty acids with branched alcohols, in particular 2-ethylhexanol, esters of C₁₈-C₃₈- alkylhydroxy carboxylic acids with linear or branched C₆-C₂₂-fatty alcohols, in particular Dioctyl Malate, esters of linear and/or branched fatty acids with polyhydric alcohols (such as, for example, propylene glycol, dimerdiol or trimertriol) and/or Guerbet alcohols, triglycerides based on C₆-C₁₀-fatty acids, liquid mono-/di-/triglyceride mixtures based on C₆-C₁₈-fatty acids, esters of C₆-C₂₂-fatty alcohols and/or Guerbet alcohols with aromatic carboxylic acids, in particular benzoic acid, esters of C₂-C₁₂-dicarboxylic acids with linear or branched alcohols having 1 to 22 carbon atoms or polyols having 2 to 10 carbon atoms and 2 to 6 hydroxyl groups, vegetable oils, branched primary alcohols, substituted cyclohexanes, linear and branched C₆-C₂₂-fatty alcohol carbonates such as, for example, Dicaprylyl Carbonate (Cetiol® CC), Guerbet carbonates, based on fatty alcohols having 6 to 18 carbon atoms, preferably 8 to 10 carbon atoms, esters of benzoic acid with linear and/or branched C₆-C₂₂-alcohols (e.g. Finsolv® TN), linear or branched, symmetrical or asymmetrical dialkyl ethers having 6 to 22 carbon atoms per alkyl group such as, for example, dicaprylyl ether (Cetiol® OE), ring-opening products of epoxidized fatty acid esters with polyols, silicone oils (cyclomethicones, silicone methicone grades, etc.) and/or aliphatic or naphthenic hydrocarbons such as, for example, squalane, squalene or dialkylcyclohexanes.

### Emulsifiers

Other surfactants may also be added to the preparations as emulsifiers, including, for example:
- products of the addition of 2 to 30 mol ethylene oxide and/or 0 to 5 mol propylene oxide onto linear C₈₋₂₂ fatty alcohols, onto C₁₂₋₂₂ fatty acids and onto alkyl phenols containing 8 to 15 carbon atoms in the alkyl group;
- C_{12/18} fatty acid monoesters and diesters of addition products of 1 to 30 mol ethylene oxide onto glycerol;
- glycerol mono- and diesters and sorbitan mono- and diesters of saturated and unsaturated fatty acids containing 6 to 22 carbon atoms and ethylene oxide addition products thereof;
- addition products of 15 to 60 mol ethylene oxide onto castor oil and/or hydrogenated castor oil;
- polyol esters and, in particular, polyglycerol esters such as, for example, polyglycerol polyricinoleate, polyglycerol poly-12-hydroxystearate or polyglycerol dimerate isostearate. Mixtures of compounds from several of these classes are also suitable;
- addition products of 2 to 15 mol ethylene oxide onto castor oil and/or hydrogenated castor oil;
- partial esters based on linear, branched, unsaturated or saturated C_{6/22} fatty acids, ricinoleic acid and 12-hydroxystearic acid and glycerol, polyglycerol, pentaerythritol, -dipentaerythritol, sugar alcohols (for example sorbitol), alkyl glucosides (for example methyl glucoside, butyl glucoside, lauryl glucoside) and polyglucosides (for example cellulose);
- mono-, di and trialkyl phosphates and mono-, di- and/or tri-PEG-alkyl phosphates and salts thereof;
- wool wax alcohols;
- polysiloxane/polyalkyl polyether copolymers and corresponding derivatives;
- mixed esters of pentaerythritol, fatty acids, citric acid and fatty alcohol and/or mixed esters of C₆₋₂₂ fatty acids, methyl glucose and polyols, preferably glycerol or polyglycerol,
- polyalkylene glycols and
- glycerol carbonate.

The addition products of ethylene oxide and/or propylene oxide onto fatty alcohols, fatty acids, alkylphenols, glycerol mono- and diesters and sorbitan mono- and diesters of fatty acids or onto castor oil are known commercially available products. They are homolog mixtures of which the average degree of alkoxylation corresponds to the ratio between the quantities of ethylene oxide and/or propylene oxide and substrate with which the addition reaction is carried out. C_{12/18} fatty acid monoesters and diesters of addition products of ethylene oxide onto glycerol are known as lipid layer enhancers for cosmetic formulations. The preferred emulsifiers are described in more detail as follows:

### • Partial glycerides

Typical examples of suitable partial glycerides are hydroxystearic acid monoglyceride, hydroxystearic acid diglyceride, isostearic acid monoglyceride, isostearic acid diglyceride, oleic acid monoglyceride, oleic acid diglyceride, ricinoleic acid monoglyceride, ricinoleic acid diglyceride, linoleic acid monoglyceride, linoleic acid diglyceride, linolenic acid monoglyceride, linolenic acid diglyceride, erucic acid monoglyceride, erucic acid diglyceride, tartaric acid monoglyceride, tartaric acid diglyceride, citric acid monoglyceride, citric acid diglyceride, malic acid monoglyceride, malic acid diglyceride and technical mixtures thereof which may still contain small quantities of triglyceride from the production process. Addition products of 1 to 30 and preferably 5 to 10 mol ethylene oxide onto the partial glycerides mentioned are also suitable.

### • Sorbitan esters

Suitable sorbitan esters are sorbitan monoisostearate, sorbitan sesquiisostearate, sorbitan diisostearate, sorbitan triisostearate, sorbitan monooleate, sorbitan sesquioleate, sorbitan dioleate, sorbitan trioleate, sorbitan monoerucate, sorbitan sesquierucate, sorbitan dierucate, sorbitan trierucate, sorbitan monoricinoleate, sorbitan sesquiricinoleate, sorbitan diricinoleate, sorbitan triricinoleate, sorbitan monohydroxystearate, sorbitan sesquihydroxystearate, sorbitan dihydroxystearate, sorbitan trihydroxystearate, sorbitan monotartrate, sorbitan sesquitartrate, sorbitan ditartrate, sorbitan tritartrate, sorbitan monocitrate, sorbitan sesquicitrate, sorbitan dicitrate, sorbitan tricitrate, sorbitan monomaleate, sorbitan sesquimaleate, sorbitan dimaleate, sorbitan trimaleate and technical mixtures thereof. Addition products of 1 to 30, and preferably 5 to 10 mol, ethylene oxide onto the sorbitan esters mentioned are also suitable.

### • Polyglycerol esters

Typical examples of suitable polyglycerol esters are Polyglyceryl-2 Dipolyhydroxystearate (Dehymuls^{®} PGPH), Polyglycerin-3-Diisostearate (Lameform^{®} TGI), Polyglyceryl-4 Isostearate (Isolan^{®} GI 34), Polyglyceryl-3 Oleate, Diisostearoyl Polyglyceryl-3 Diisostearate (Isolan^{®} PDI), Polyglyceryl-3 Methylglucose Distearate (Tego Care^{®} 450), Polyglyceryl-3 Beeswax (Cera Bellina^{®}, Polyglyceryl-4 Caprate (Polyglycerol Caprate T2010/90), Polyglyceryl-3 Cetyl Ether (Chimexane^{®} NL), Polyglyceryl-3 Distearate (Cremophor® GS 32) and Polyglyceryl Polyricinoleate (Admul^{®} WOL 1403), Polyglyceryl Dimerate Isostearate and mixtures thereof. Examples of other suitable polyolesters are the mono-, di- and triesters of trimethylol propane or pentaerythritol with lauric acid, cocofatty acid, tallow fatty acid, palmitic acid, stearic acid, oleic acid, behenic acid and the like optionally reacted with 1 to 30 mol ethylene oxide.

Typical anionic emulsifiers are aliphatic C₁₂₋₂₂ fatty acids such as palmitic acid, stearic acid or behenic acid, for example, and C₁₂₋₂₂ dicarboxylic acids such as azelaic acid or sebacic acid, for example.

Other suitable emulsifiers are zwitterionic surfactants. Zwitterionic surfactants are surface-active compounds which contain at least one quaternary ammonium group and at least one carboxylate and one sulphonate group in the molecule. Particularly suitable zwitterionic surfactants are the so-called betaines such as the N-alkyl-N,N-dimethyl ammonium glycinates, for example cocoalkyl dimethyl ammonium glycinate, N-acylaminopropyl-N,N-dimethyl ammonium glycinates, for example cocoacylaminopropyl dimethyl ammonium glycinate, and 2-alkyl-3-carboxymethyl-3-hydroxyethyl imidazolines containing 8 to 18 carbon atoms in the alkyl or acyl group and cocoacylaminoethyl hydroxyethyl carboxymethyl glycinate. The fatty acid amide derivative known under the CTFA name of *Cocamidopropyl Betaine* is particularly preferred. Ampholytic surfactants are also suitable emulsifiers. Ampholytic surfactants are surface-active compounds which, in addition to a C_{8/18} alkyl or acyl group, contain at least one free amino group and at least one -COOH- or -SO₃H- group in the molecule and which are capable of forming inner salts. Examples of suitable ampholytic surfactants are N-alkyl glycines, N-alkyl propionic acids, N-alkylaminobutyric acids, N-alkyliminodipropionic acids, N-hydroxyethyl-N-alkylamidopropyl glycines, N-alkyl taurines, N-alkyl sarcosines, 2-alkylaminopropionic acids and alkylaminoacetic acids containing around 8 to 18 carbon atoms in the alkyl group. Particularly preferred ampholytic surfactants are N-cocoalkylaminopropionate, cocoacylaminoethyl aminopropionate and C_{12/18} acyl sarcosine.

### Superfatting agents

Superfatting agents may be selected from substances such as, for example, lanolin and lecithin and also polyethoxylated or acylated lanolin and lecithin derivatives, polyol fatty acid esters, monoglycerides and fatty acid alkanolamides, the fatty acid alkanolamides also serving as foam stabilizers.

### Consistency factors

The consistency factors mainly used are fatty alcohols or hydroxyfatty alcohols containing 12 to 22 carbon atoms, preferably 16 to 18 carbon atoms, and also partial glycerides, fatty acids or hydroxyfatty acids. A combination of these substances with alkyl oligoglucosides and/or fatty acid N-methyl glucamides of the same chain length and/or polyglycerol poly-12-hydroxystearates is preferably used.

### Thickening agents

Suitable thickeners are polymeric thickeners such as Aerosil® types (hydrophilic silicas), polysaccharides, more especially xanthan gum, guar-guar, agar-agar, alginates and tyloses, carboxymethyl cellulose and hydroxyethyl cellulose, also relatively high molecular weight polyethylene glycol monoesters and diesters of fatty acids, polyacrylates (for example Carbopols® [Goodrich] or Synthalens® [Sigma]), polyacrylamides, polyvinyl alcohol and polyvinyl pyrrolidone, surfactants such as, for example, ethoxylated fatty acid glycerides, esters of fatty acids with polyols, for example pentaerythritol or trimethylol propane, narrow-range fatty alcohol ethoxylates and electrolytes such as sodium chloride and ammonium chloride.

### Polymers

Suitable cationic polymers are, for example, cationic cellulose derivatives such as, for example, the quaternised hydroxyethyl cellulose obtainable from Amerchol under the name of Polymer JR 400®, cationic starch, copolymers of diallyl ammonium salts and acrylamides, quaternised vinyl pyrrolidone/vinyl imidazole polymers such as, for example, Luviquat® (BASF), condensation products of polyglycols and amines, quaternised collagen polypeptides such as, for example, Lauryldimonium Hydroxypropyl Hydrolyzed Collagen (Lamequat® L, Grünau), quaternised wheat polypeptides, polyethyleneimine, cationic silicone polymers such as, for example, amodimethicone, copolymers of adipic acid and dimethylaminohydroxypropyl diethylenetriamine (Cartaretine^{®}, Sandoz), copolymers of acrylic acid with dimethyl diallyl ammonium chloride (Merquat^{®} 550, Chemviron), polyaminopolyamides and crosslinked water-soluble polymers thereof, cationic chitin derivatives such as, for example, quaternised chitosan, optionally in microcrystalline distribution, condensation products of dihaloalkyls, for example dibromobutane, with bis-dialkylamines, for example bis-dimethylamino-1,3-propane, cationic guar gum such as, for example, Jaguar^{®}CBS, Jaguar^{®}C-17, Jaguar^{®}C-16 of Celanese, quaternised ammonium salt polymers such as, for example, Mirapol^{®} A-15, Mirapol^{®} AD-1, Mirapol^{®} AZ-1 of Miranol and the various polyquaternium types (for example 6, 7, 32 or 37) which can be found in the market under the tradenames Rheocare^{®} CC or Ultragel^{®} 300.

Suitable anionic, zwitterionic, amphoteric and nonionic polymers are, for example, vinyl acetate/crotonic acid copolymers, vinyl pyrrolidone/vinyl acrylate copolymers, vinyl acetate/butyl maleate/isobornyl acrylate copolymers, methyl vinylether/maleic anhydride copolymers and esters thereof, uncrosslinked and polyol-crosslinked polyacrylic acids, acrylamidopropyl trimethylammonium chloride/acrylate copolymers, octylacrylamide/methyl methacrylate/tert.-butylaminoethyl methacrylate/2-hydroxypropyl methacrylate copolymers, polyvinyl pyrrolidone, vinyl pyrrolidone/vinyl acetate copolymers, vinyl pyrrolidone/dimethylaminoethyl methacrylate/vinyl caprolactam terpolymers and optionally derivatized cellulose ethers and silicones.

### Pearlising waxes

Suitable pearlising waxes are, for example, alkylene glycol esters, especially ethylene glycol distearate; fatty acid alkanolamides, especially cocofatty acid diethanolamide; partial glycerides, especially stearic acid monoglyceride; esters of polybasic, optionally hydroxysubstituted carboxylic acids with fatty alcohols containing 6 to 22 carbon atoms, especially long-chain esters of tartaric acid; fatty compounds such as, for example, fatty alcohols, fatty ketones, fatty aldehydes, fatty ethers and fatty carbonates which contain in all at least 24 carbon atoms, especially laurone and distearylether; fatty acids such as stearic acid, hydroxystearic acid or behenic acid, ring opening products of olefin epoxides containing 12 to 22 carbon atoms with fatty alcohols containing 12 to 22 carbon atoms and/or polyols containing 2 to 15 carbon atoms and 2 to 10 hydroxyl groups and mixtures thereof.

### Silicones

Suitable silicone compounds are, for example, dimethyl polysiloxanes, methylphenyl polysiloxanes, cyclic silicones and amino-, fatty acid-, alcohol-, polyether-, epoxy-, fluorine-, glycoside- and/or alkyl-modified silicone compounds which may be both liquid and resin-like at room temperature. Other suitable silicone compounds are simethicones which are mixtures of dimethicones with an average chain length of 200 to 300 dimethylsiloxane units and hydrogenated silicates. A detailed overview of suitable volatile silicones can be found in Todd et al. in Cosm. Toil. 91, 27 (1976**).**

### Waxes

Besides natural oils used, waxes may also be present in the preparations, more especially natural waxes such as, for example, candelilla wax, carnauba wax, Japan wax, espartograss wax, cork wax, guaruma wax, rice oil wax, sugar cane wax, ouricury wax, montan wax, beeswax, shellac wax, spermaceti, lanolin (wool wax), uropygial fat, ceresine, ozocerite (earth wax), petrolatum, paraffin waxes and microwaxes; chemically modified waxes (hard waxes) such as, for example, montan ester waxes, sasol waxes, hydrogenated jojoba waxes and synthetic waxes such as, for example, polyalkylene waxes and polyethylene glycol waxes.

### Stabilizers

Metal salts of fatty acids such as, for example, magnesium, aluminium and/or zinc stearate or ricinoleate may be used as stabilizers.

### Primary sun protection factors

Primary sun protection factors in the context of the invention are, for example, organic substances (light filters) which are liquid or crystalline at room temperature and which are capable of absorbing ultraviolet radiation and of releasing the energy absorbed in the form of longer-wave radiation, for example heat. UV-B filters can be oil-soluble or water-soluble. The following are examples of oil-soluble substances:
- 3-benzylidene camphor or 3-benzylidene norcamphor and derivatives thereof, for example 3-(4-methylbenzylidene)-camphor;
- 4-aminobenzoic acid derivatives, preferably 4-(dimethylamino)benzoic acid-2-ethylhexyl ester, 4-(dimethylamino)-benzoic acid-2-octyl ester and 4-(dimethylamino)benzoic acid amyl ester;
- esters of cinnamic acid, preferably 4-methoxycinnamic acid-2-ethylhexyl ester, 4-methoxycinnamic acid propyl ester, 4-methoxycinnamic acid isoamyl ester, 2-cyano-3,3-phenylcinnamic acid-2-ethylhexyl ester (Octocrylene);
- esters of salicylic acid, preferably salicylic acid-2-ethylhexyl ester, salicylic acid-4-isopropylbenzyl ester, salicylic acid homomenthyl ester;
- derivatives of benzophenone, preferably 2-hydroxy-4-methoxybenzophenone, 2-hydroxy-4-methoxy-4'-methylbenzophenone, 2,2'-dihydroxy-4-methoxybenzophenone;
- esters of benzalmalonic acid, preferably 4-methoxybenzalmalonic acid di-2-ethylhexyl ester;
- triazine derivatives such as, for example, 2,4,6-trianilino-(p-carbo-2'-ethyl-1'-hexyloxy)-1,3,5-triazine and Octyl Triazone or Dioctyl Butamido Triazone (Uvasorb^{®} HEB);
- propane-1,3-diones such as, for example, 1-(4-tert.butylphenyl)-3-(4'-methoxyphenyl)-propane-1,3-dione;
- ketotricyclo(5.2.1.0)decane derivatives.

Suitable water-soluble substances are
- 2-phenylbenzimidazole-5-sulphonic acid and alkali metal, alkaline earth metal, ammonium, alkylammonium, alkanolammonium and glucammonium salts thereof;
- sulphonic acid derivatives of benzophenones, preferably 2-hydroxy-4-methoxybenzophenone-5-sulphonic acid and salts thereof;
- sulphonic acid derivatives of 3-benzylidene camphor such as, for example, 4-(2-oxo-3-bomylidenemethyl)-benzene sulphonic acid and 2-methyl-5-(2-oxo-3-bomylidene)-sulphonic acid and salts thereof.

Typical UV-A filters are, in particular, derivatives of benzoyl methane such as, for example, 1-(4'-tert.butylphenyl)-3-(4'-methoxyphenyl)-propane-1,3-dione, 4-tert.butyl-4'-methoxydibenzoyl methane (Parsol^{®} 1789) or 1-phenyl-3-(4'-isopropylphenyl)-propane-1,3-dione and the enamine compounds (BASF). The UV-A and UV-B filters may of course also be used in the form of mixtures. Particularly favourable combinations consist of the derivatives of benzoyl methane, for example 4-tert.butyl-4'-methoxydibenzoyl methane (Parsol^{®} 1789) and 2-cyano-3,3-phenylcinnamic acid-2-ethylhexyl ester (Octocrylene^{®}, in combination with esters of cinnamic acid, preferably 4-methoxycinnamic acid-2-ethylhexyl ester and/or 4-methoxycinnamic acid propyl ester and/or 4-methoxycinnamic acid isoamyl ester. Combinations such as these are advantageously combined with water-soluble filters such as, for example, 2-phenylbenzimidazole-5-sulphonic acid and alkali metal, alkaline earth metal, ammonium, alkylammonium, alkanolammonium and glucammonium salts thereof.

### Secondary sun protection factors

Besides the groups of primary sun protection factors mentioned above, secondary sun protection factors of the antioxidant type may also be used. Secondary sun protection factors of the antioxidant type interrupt the photochemical reaction chain which is initiated when UV rays penetrate into the skin. Typical examples are amino acids (for example glycine, histidine, tyrosine, tryptophane) and derivatives thereof, imidazoles (for example urocanic acid) and derivatives thereof, peptides such as D,L-carnosine, D-carnosine, L-carnosine and derivatives thereof (for example anserine), carotinoids, carotenes (for example alpha-carotene, beta-carotene, lycopene) and derivatives thereof, chlorogenic acid and derivatives thereof, liponic acid and derivatives thereof (for example dihydroliponic acid), aurothioglucose, propylthiouracil and other thiols (for example thioredoxine, glutathione, cysteine, cystine, cystamine and glycosyl, N-acetyl, methyl, ethyl, propyl, amyl, butyl and lauryl, palmitoyl, oleyl, alphalinoleyl, cholesteryl and glyceryl esters thereof) and their salts, dilaurylthiodipropionate, distearylthiodipropionate, thiodipropionic acid and derivatives thereof (esters, ethers, peptides, lipids, nucleotides, nucleosides and salts) and sulphoximine compounds (for example butionine sulphoximines, homocysteine sulphoximine, butionine sulphones, penta-, hexa- and heptathionine sulphoximine) in very small compatible dosages, also (metal) chelators (for example alpha-hydroxyfatty acids, palmitic acid, phytic acid, lactoferrine), alpha-hydroxy acids (for example citric acid, lactic acid, malic acid), humic acid, bile acid, bile extracts, bilirubin, biliverdin, EDTA, EGTA and derivatives thereof, unsaturated fatty acids and derivatives thereof (for example linoleic acid, oleic acid), folic acid and derivatives thereof, ubiquinone and ubiquinol and derivatives thereof, vitamin C and derivatives thereof (for example ascorbyl palmitate, Mg ascorbyl phosphate, ascorbyl acetate), tocopherols and derivatives (for example vitamin E acetate), vitamin A and derivatives (vitamin A palmitate) and coniferyl benzoate of benzoin resin, rutinic acid and derivatives thereof, glycosyl rutin, ferulic acid, furfurylidene glucitol, carnosine, butyl hydroxytoluene, butyl hydroxyanisole, nordihydroguaiac resin acid, nordihydroguaiaretic acid, trihydroxybutyrophenone, uric acid and derivatives thereof, mannose and derivatives thereof, superoxide dismutase, titanium dioxide (for example dispersions in ethanol), zinc and derivatives thereof (for example ZnO, ZnSO₄), selenium and derivatives thereof (for example selenium methionine), stilbenes and derivatives thereof (for example stilbene oxide, trans-stilbene oxide) and derivatives of these active substances like salts, esters, ethers, sugars, nucleotides, nucleosides, peptides and lipids.

### Biogenic agents

In the context of the invention, biogenic agents are, for example, tocopherol, tocopherol acetate, tocopherol palmitate, ascorbic acid, (deoxy)ribonucleic acid and fragmentation products thereof, β-glucans, retinol, bisabolol, allantoin, phytantriol, panthenol, AHA acids, amino acids, ceramides, pseudoceramides, essential oils, plant extracts, for example prune extract, bambara nut extract, and vitamin complexes.

### Anti-microbial agents

Suitable anti-microbial agents are in principle all substances effective against Gram-positive bacteria such as, for example, 4- hydroxybenzoic acid and its salts and esters, N-(4-chlorophenyl)-N'-(3,4- dichlorophenyl)urea, 2,4,4'-trichloro-2'-hydroxy-diphenyl ether (triclosan), 4-chloro-3,5-dimethyl-phenol, 2,2'-methylenebis(6-bromo-4- chlorophenol), 3-methyl-4-(1-methylethyl)phenol, 2-benzyl-4-chloro-phenol, 3-(4-chlorophenoxy)-1,2-propanediol, 3-iodo-2-propynyl butylcarbamate, chlorhexidine, 3,4,4'-trichlorocarbanilide (TTC), antibacterial fragrances, thymol, thyme oil, eugenol, oil of cloves, menthol, mint oil, farnesol, phenoxyethanol, glycerol monocaprate, glycerol monocaprylate, glycerol monolaurate (GML), diglycerol monocaprate (DMC), salicylic acid N-alkylamides such as, for example, n-octylsalicylamide or n- decylsalicylamide.

### Enzyme inhibitors

Suitable enzyme inhibitors are, for example, esterase inhibitors. These are preferably trialkyl citrates such as trimethyl citrate, tripropyl citrate, triisopropyl citrate, tributyl citrate and, in particular, triethyl citrate (Hydagen CAT). The substances inhibit enzyme activity, thereby reducing the formation of odour. Other substances which are suitable esterase inhibitors are sterol sulphates or phosphates such as, for example, lanosterol, cholesterol, campesterol, stigmasterol and sitosterol sulphate or phosphate, dicarboxylic acids and esters thereof such as, for example, glutaric acid, monoethyl glutarate, diethyl glutarate, adipic acid, monoethyl adipate, diethyl adipate, malonic acid and diethyl malonate, hydroxycarboxylic acids and esters thereof such as, for example, citric acid, malic acid, tartaric acid or diethyl tartrate, and zinc glycinate.

### Film formers

Standard film formers are, for example, chitosan, microcrystalline chitosan, quaternised chitosan, polyvinyl pyrrolidone, vinyl pyrrolidone/vinyl acetate copolymers, polymers of the acrylic acid series, quaternary cellulose derivatives, collagen, hyaluronic acid and salts thereof, and similar compounds.

### Anti-dandruff agents

Suitable anti-dandruff agents are Pirocton Olamin (1-hydroxy-4-methyl-6-(2,4,4-trimethylpentyl)-2-(1H)-pyridinone monoethanolamine salt), Baypival^{®} (Climbazole), Ketoconazol^{®} (4-acetyl-1-{4-[2-(2,4-dichlorophenyl) r-2-(1H-imidazol-1-ylmethyl)-1,3-dioxylan-c-4-yl-methoxyphenyl}-piperazine, ketoconazole, elubiol, selenium disulphide, colloidal sulphur, sulphur polyethylene glycol sorbitan monooleate, sulphur ricinol polyethoxylate, sulphur tar distillate, salicylic acid (or in combination with hexachlorophene), undecylenic acid, monoethanolamide sulphosuccinate Na salt, Lamepon^{®} UD (protein/undecylenic acid condensate), zinc pyrithione, aluminium pyrithione and magnesium pyrithione/dipyrithione magnesium sulphate.

### Hydrotropes

In addition, hydrotropes, for example ethanol, isopropyl alcohol or polyols, may be used to improve flow behaviour. Suitable polyols preferably contain 2 to 15 carbon atoms and at least two hydroxyl groups. The polyols may contain other functional groups, more especially amino groups, or may be modified with nitrogen. Typical examples are
- glycerol;
- alkylene glycols such as, for example, ethylene glycol, diethylene glycol, propylene glycol, butylene glycol, hexylene glycol and polyethylene glycols with an average molecular weight of 100 to 1000 Dalton;
- technical oligoglycerol mixtures with a degree of self-condensation of 1.5 to 10 such as, for example, technical diglycerol mixtures with a diglycerol content of 40 to 50% by weight;
- methylol compounds such as, in particular, trimethylol ethane, trimethylol propane, trimethylol butane, pentaerythritol and dipentaerythritol;
- lower alkyl glucosides, particularly those containing 1 to 8 carbon atoms in the alkyl group, for example methyl and butyl glucoside;
- sugar alcohols containing 5 to 12 carbon atoms, for example sorbitol or mannitol,
- sugars containing 5 to 12 carbon atoms, for example glucose or sucrose;
- amino sugars, for example glucamine;
- dialcoholamines such as diethanolamine or 2-aminopropane-1,3-diol.

### Preservatives

Suitable preservatives are, for example, phenoxyethanol, formaldehyde solution, parabens, pentanediol or sorbic acid and the other classes of compounds listed in Appendix 6, Parts A and B of the Kosmetikverordnung ("Cosmetics Directive").

### Complexing agents

The complexing agents used may be selected from EDTA, NTA, phosphonic acids, Triton B, turpinal and phenacetin. In addition, reducing agents such as, for example, ascorbic acid, sodium sulphate, sodium thiosulphate and the like may be present. Suitable alkalizing agents are ammonia, monoethanolamines, (L) arginine, AMP, etc.

### Perfume oils

Suitable perfume oils are mixtures of natural and synthetic perfumes. Natural perfumes include the extracts of blossoms (lily, lavender, rose, jasmine, neroli, ylang-ylang), stems and leaves (geranium, patchouli, petitgrain), fruits (anise, coriander, caraway, juniper), fruit peel (bergamot, lemon, orange), roots (nutmeg, angelica, celery, cardamom, costus, iris, calmus), woods (pinewood, sandalwood, guaiac wood, cedarwood, rosewood), herbs and grasses (tarragon, lemon grass, sage, thyme), needles and branches (spruce, fir, pine, dwarf pine), resins and balsams (galbanum, elemi, benzoin, myrrh, olibanum, opoponax). Animal raw materials, for example civet and beaver, may also be used. Typical synthetic perfume compounds are products of the ester, ether, aldehyde, ketone, alcohol and hydrocarbon type. Examples of perfume compounds of the ester type are benzyl acetate, phenoxyethyl isobutyrate, p-tert.butyl cyclohexylacetate, linalyl acetate, dimethyl benzyl carbinyl acetate, phenyl ethyl acetate, linalyl benzoate, benzyl formate, ethylmethyl phenyl glycinate, allyl cyclohexyl propionate, styrallyl propionate and benzyl salicylate. Ethers include, for example, benzyl ethyl ether while aldehydes include, for example, the linear alkanals containing 8 to 18 carbon atoms, citral, citronellal, citronellyloxyacetaldehyde, cyclamen aldehyde, hydroxycitronellal, lilial and bourgeonal. Examples of suitable ketones are the ionones, □-isomethylionone and methyl cedryl ketone. Suitable alcohols are anethol, citronellol, eugenol, isoeugenol, geraniol, linalool, phenylethyl alcohol and terpineol. The hydrocarbons mainly include the terpenes and balsams. However, it is preferred to use mixtures of different perfume compounds which, together, produce an agreeable perfume. Other suitable perfume oils are essential oils of relatively low volatility which are mostly used as aroma components. Examples are sage oil, camomile oil, clove oil, melissa oil, mint oil, cinnamon leaf oil, lime-blossom oil, juniper berry oil, vetiver oil, olibanum oil, galbanum oil, ladanum oil and lavendin oil. The following are preferably used either individually or in the form of mixtures: bergamot oil, dihydromyrcenol, lilial, lyral, citronellol, phenylethyl alcohol, hexylcinnamaldehyde, geraniol, benzyl acetone, cyclamen aldehyde, linalool, Boisambrene Forte, Ambroxan, indole, hedione, sandelice, citrus oil, mandarin oil, orange oil, allylamyl glycolate, cyclovertal, lavendin oil, clary oil, damascone, geranium oil bourbon, cyclohexyl salicylate, Vertofix Coeur, Iso-E-Super, Fixolide NP, evernyl, iraldein gamma, phenylacetic acid, geranyl acetate, benzyl acetate, rose oxide, romillat, irotyl and floramat.

### Dyes

Suitable dyes are any of the substances suitable and approved for cosmetic purposes as listed, for example, in the publication **"**Kosmetische Färbemittel" of the Farbstoffkommission der Deutschen Forschungsgemeinschaft, Verlag Chemie, Weinheim, 1984, pages 81 to 106**.** Examples include cochineal red A (C.I. 16255), patent blue V (C.I. 42051), indigotin (C.I. 73015), chlorophyllin (C.I. 75810), quinoline yellow (C.I. 47005), titanium dioxide (C.I. 77891), indanthrene blue RS (C.I. 69800) and madder lake (C.I. 58000). Luminol may also be present as a luminescent dye. These dyes are normally used in concentrations of 0.001 to 0.1% by weight, based on the mixture as a whole.

The total percentage content of auxiliaries and additives may be from 1 to 50% by weight and is preferably from 5 to 40% by weight, based on the particular composition. The compositions may be produced by standard hot or cold processes.

### Examples

### Manufacturing Example M1

151 g (1.05 moles) caprylic acid, 298 g (1.05 moles) stearic acid, 219 g (1.5 moles) of adipic acid and 0.3 g of hypophosphoric acid were introduced into a stirred reactor and heated to 70 °C under a reduced pressure of 20 mbar. 447 g (3 moles) of triethanolamine were then added dropwise in portions and at the same time temperature was increased to 120 °C. After the addition, the reaction mixture was heated to 160 °C, pressure was reduced to 3 mbar and the mixture was stirred under these conditions for 2.5 h until the acid value had fallen to below 5 mg KOH/g. The mixture was then cooled to 60 °C, the vacuum was broken by introduction of nitrogen, and 0.6 g of hydrogen peroxide was added in the form of a 30% by weight aqueous solution. For the quaternisation step, the resulting ester was dissolved in 376 g of isopropyl alcohol, and 357 g (2.83 moles) of dimethyl sulphate were added to the resulting solution over a period of 1 hour at such a rate that the temperature did not rise above 65 °C. After the addition, the mixture was stirred for another 2.5 h, the total nitrogen content being regularly checked by sampling. The reaction was terminated when constant total nitrogen content was reached. A product with a solids content of 80 % b.w. was obtained.

### Comparison Example C1

567 g (2.1 moles) of partly hydrogenated palm oil fatty acid, 219 g (1.5 moles) of adipic acid and 0.3 g of hypophosphoric acid were introduced into a stirred reactor and heated to 70 °C under a reduced pressure of 20 mbar. 447 g (3 moles) of triethanolamine were then added dropwise in portions and, at the same time, the temperature was increased to 120 °C. After the addition, the reaction mixture was heated to 160 °C, the pressure was reduced to 3 mbar and the mixture was stirred under these conditions for 2.5 h until the acid value had fallen to below 5 mg KOH/g. The mixture was then cooled to 60 °C, the vacuum was broken by introduction of nitrogen, and 0.6 g of hydrogen peroxide was added in the form of a 30% by weight aqueous solution. For the quaternisation step, the resulting ester was dissolved in 376 g of isopropyl alcohol, and 357 g (2.83 moles) of dimethyl sulphate were added to the resulting solution over a period of 1 hour at such a rate that the temperature did not rise above 65 °C. After the addition, the mixture was stirred for another 2.5 h, the total nitrogen content being regularly checked by sampling. The reaction was terminated when constant total nitrogen content had been reached. A product with a solids content of 80 % b.w. was obtained.

### Determination of viscosity

The polymeric esterquats according to inventive example M1 and comparison example C1 as well as a non-polymeric esterquat ("C2" = Dehyquart^{®} AU 46, Cognis Iberia, Spain) were incorporated into an acidic hair care preparation consisting of 6 % b.w. surfactant, 1 % b.w. cetearyl alcohol and 1 % b.w. non-ionic emulsifer (Eumulgin^{®} B2, Cognis Deutschland GmbH & Co. KG) (water added to 100 % b.w.). The pH value of the formulations was adjusted to 3.5 while the formation of the emulsion was achieved by lightly stirring the mixtures at room temperature. In all cases, homogenous emulsions were prepared. The viscosity of the products was determined according to the Brookfield method (RVT, 20 °C, 10 rpm, Spindle 1). The results are compiled in Table 1. As one can see, the stability of the emulsions using the polymeric esterquat according to the present invention was much better compared to the polymeric esterquat with symmetric side chains and comparable to the results achieved by using a standard cationic surfactant.

**Table 1**

| **Viscosities** | | | | |
|---|---|---|---|---|
| **Example** | **Esterquat** | **Viscosity [mPas]** | | |
| | | after 1 d | after 2 d | after 10 d |
| 1 | M1 | 9.500 | 9.400 | 9.000 |
| C1 | C1 | 9.300 | 8.900 | 7.000 |
| C2 | C2 | 9.500 | 9.500 | 9.100 |

### Softening and anti-static properties

For testing the softening and anti-static properties, the polymeric esterquats according to the present invention, the comparative polymeric esterquats and the monomeric cationic surfactant were diluted with water to give 5 % aqueous solutions. The tests were conducted using strands of brown hair (Alkinco #6634, length 12 cm, weight 1 g). In order to determine the *wet combability,* the strands were tested before and after treatment with 100 ml of the test solutions over a period of 5 min. Subsequently, the strands were washed over a period of 1 minute with 1 l water of elevated temperature (about 38 °C). The results are compiled in Table 2; the test method is described in detail in J. Soc. Cosm. Chem. 24, 782 (1973**).** The *softening* properties were determined by a panel of 6 trained people. The results, also compiled in Table 2, represent the average values from three test cycles. The lower the numbers, the better is the softness of the strands. As one can see, the new polymeric esterquats with asymmetric side chains exhibit an improved softening behaviour and reduces the force for wet combing more than the corresponding polymeric esterquats with symmetric side chains does.

**Table 2**

| **Softening and wet combability** | | | | | |
|---|---|---|---|---|---|
| **Example** | **Esterquat** | **Softening** | **Wet combability [mJ]** | | |
| | | | prior to treatment | after treatment | Difference |
| 2 | M1 | 1,7 | 77.4 | 22.6 | 54.8 |
| C3 | C1 | 1,9 | 69.6 | 23.4 | 46.2 |
| C4 | C2 | 2,1 | 63.5 | 20.4 | 43.1 |

In the following Table 3 some formulation examples are given.

**Table 3**

| **Cosmetic compositions (Water and preservatives adding to 100 % b.w.)** | | | | | | | | | | |
|---|---|---|---|---|---|---|---|---|---|---|
| **Composition (INCI)** | **1** | **2** | **3** | **4** | **5** | **6** | **7** | **8** | **9** | **10** |
| **Texapon® NSO** Sodium Laureth Sulphate | - | - | - | - | - | - | 38.0 | 38.0 | 25.0 | - |
| **Texapon® SB 3** Disodium Laureth Sulphosuccinate | - | - | - | - | - | - | - | - | 10.0 | - |
| **Plantacare® 818** Coco Glucosides | - | - | - | - | - | - | 7.0 | 7.0 | 6.0 | - |
| **Plantacare® PS 10** Sodium Laureth Sulphate (and) Coco Glucosides | - | - | - | - | - | - | - | - | - | 16.0 |
| **Dehyton® PK 45** Cocamidopropyl Betaine | - | - | - | - | - | - | - | - | 10.0 | - |
| **Polymeric Esterquat according to Example M1** | 2.0 | 2.0 | 2.0 | 2.0 | 4.0 | 4.0 | 2.0 | 2.0 | 2.0 | 2.0 |
| **Dehyquart L® 80** Dicocoylmethylethoxymonium Methosulphate (and) Propylenglycol | 1.2 | 1.2 | 1.2 | 1.2 | 0.6 | 0.6 | - | - | - | - |
| **Eumulgin® B2** Ceteareth-20 | 0.8 | 0.8 | - | 0.8 | - | 1.0 | - | - | - | - |
| **Eumulgin® VL 75** Lauryl Glucoside (and) Polyglyceryl-2 Polyhydroxystearate (and) Glycerin | - | - | 0.8 | - | 0.8 | - | - | - | - | - |
| **Lanette® O** Cetearyl Alcohol | 2.5 | 2.5 | 2.5 | 2.5 | 3.0 | 2.5 | - | - | - | - |
| **Cutina® GMS** Glyceryl Stearate | 0.5 | 0.5 | 0.5 | 0.5 | 0.5 | 1.0 | - | - | - | - |
| **Cetiol**® **HE** PEG-7 Glyceryl Cocoate | 1.0 | - | - | - | - | - | - | - | 1.0 | |
| **Cetiol® PGL** Hexyldecanol (and) Hexyldecyl Laurate | - | 1.0 | - | - | 1.0 | - | - | - | - | - |
| **Cetiol® V** Decyl Oleate | - | - | - | 1.0 | - | - | - | - | - | - |
| **Eutanol® G** Octyldodecanol | - | - | 1.0 | - | - | 1.0 | - | - | - | - |
| **Nutrilan® Keratin W** Hydrolyzed Keratin | - | - | - | 2.0 | - | - | - | - | - | - |
| **Lamesoft® LMG** Glyceryl Laurate (and) Potassium Cocoyl Hydrolyzed Collagen | - | - | - | - | - | - | 3.0 | 2.0 | 4.0 | - |
| **Euperlan® PK 3000 AM** Glycol Distearate (and) Laureth-4 (and) Cocamidopropyl Betaine | - | - | - | - | - | - | - | 3.0 | 5.0 | 5.0 |
| **Generol® 122 N** Soja Sterol | - | - | - | - | 1.0 | 1.0 | - | - | - | - |
| **Hydagen® CMF** Chitosan | 1.0 | 1.0 | 1.0 | 1.0 | 1.0 | 1.0 | 1.0 | 1.0 | 1.0 | 1.0 |
| **Copherol® 1250** Tocopherol Acetate | - | - | 0.1 | 0.1 | - | - | - | - | - | - |
| **Arlypon® F** Laureth-2 | - | - | - | - | - | - | 3.0 | 3.0 | 1.0 | - |
| **Sodium Chloride** | - | - | - | - | - | - | - | 1.5 | - | 1.5 |

| | | | | | | | | | | |
|---|---|---|---|---|---|---|---|---|---|---|
| (1-4) Hair cure, (5-6) Conditioner, (7-8) Shower bath, (9) Shower gel, (10) Washing lotion | | | | | | | | | | |

**Table 3**

| **Cosmetic compositions (Water and preservatives adding to 100 % b.w.) - Cont.** | | | | | | | | | | |
|---|---|---|---|---|---|---|---|---|---|---|
| **Composition (INCI)** | **11** | **12** | **13** | **14** | **15** | **16** | **17** | **18** | **19** | **20** |
| **Texapon® NSO** Sodium Laureth Sulphate | 20.0 | 20.0 | 12.4 | - | 25.0 | 11.0 | - | - | - | - |
| **Texapon® K 14 S** Sodium Myreth Sulphate | - | - | - | - | - | - | - | - | 11.0 | 23.0 |
| **Texapon® SB 3** Disodium Laureth Sulphosuccinate | - | - | - | - | - | 7.0 | - | - | - | - |
| **Plantacare® 818** Coco Glucosides | 5.0 | 5.0 | 4.0 | - | - | - | - | - | 6.0 | 4.0 |
| **Plantacare® 2000** Decyl Glucoside | - | - | - | - | 5.0 | 4.0 | - | - | - | - |
| **Plantacare® PS 10** Sodium Laureth Sulphate (and) Coco Glucosides | - | - | - | 40.0 | - | - | 16.0 | 17.0 | - | - |
| **Dehyton® PK 45** Cocamidopropyl Betaine | 20.0 | 20.0 | - | - | 8.0 | - | - | - | - | 7.0 |
| **Eumulgin® B1** Ceteareth-12 | - | - | - | - | 1.0 | - | - | - | - | - |
| **Eumulgin® B2** Ceteareth-20 | - | - | - | 1.0 | - | - | - | - | - | - |
| **Lameform® TGI** Polyglyceryl-3 Isostearate | - | - | - | 4.0 | - | - | - | - | - | - |
| **Dehymuls® PGPH** Polyglyceryl-2 Dipolyhydroxystearate | - | - | 1.0 | - | - | - | - | - | - | - |
| **Monomuls® 90-L** 12 Glyceryl Laurate | - | - | - | - | - | - | - | - | 1.0 | 1.0 |
| **Cetiol® HE** PEG-7 Glyceryl Cocoate | - | 0.2 | - | - | - | - | - | - | - | - |
| **Eutanol® G** Octyldodecanol | - | - | - | 3.0 | - | - | - | - | - | - |
| **Nutrilan® Keratin W** Hydrolyzed Keratin | - | - | - | - | - | - | - | - | 2.0 | 2.0 |
| **Nutrilan® I** Hydrolyzed Collagen | 1.0 | - | - | - | - | 2.0 | - | 2.0 | - | - |
| **Lamesoft® LMG** Glyceryl Laurate (and) Potassium Cocoyl Hydrolyzed Collagen | - | - | - | - | - | - | - | - | 1.0 | - |
| **Lamesoft® 156** Hydrogenated Tallow Gyceride (and) Potassium Cocoyl Hydrolyzed Collagen | - | - | - | - | - | - | - | - | - | 5.0 |
| **Gluadin® WK** Sodium Cocoyl Hydrolyzed Wheat Protein | 1.0 | 1.5 | 4.0 | 1.0 | 3.0 | 1.0 | 2.0 | 2.0 | 2.0 | - |
| **Euperlan® PK 3000 AM** Glycol Distearate (and) Laureth-4 (and) Cocamidopropyl Betaine | 5.0 | 3.0 | 4.0 | - | - | - | - | 3.0 | 3.0 | - |
| **Arlypon® F** Laureth-2 | 2.6 | 1.6 | - | 1.0 | 1.5 | - | - | - | - | - |
| **Polymeric Esterquat according to Example M1** | 2.0 | 2.0 | 2.0 | 2.0 | 4.0 | 4.0 | 2.0 | 2.0 | 2.0 | 2.0 |
| **Hydagen® CMF** Chitosan | 1.0 | 1.0 | 1.0 | 1.0 | 1.0 | 1.0 | 1.0 | 1.0 | 1.0 | 1.0 |
| **Sodium Chloride** | - | - | - | - | - | 1.6 | 2.0 | 2.2 | - | 3.0 |
| **Glycerin** (86 Gew.-%ig) | - | 5.0 | - | - | - | - | - | 1.0 | 3.0 | - |

| | | | | | | | | | | |
|---|---|---|---|---|---|---|---|---|---|---|
| (11-14) Shower bath "Two-in-One"), (15-20) Shampoo | | | | | | | | | | |

## Claims

1. Polymeric esterquats with asymmetric side chains, obtainable by reacting alkanolamines with a mixture of
(i) C₆-C₁₀ monocarboxylic acids
(ii) C₁₂-C₂₂ monocarboxylic acids, and
(iii) Dicarboxylic acids, and
quaternising the resulting esters with alkylation agents, optionally after alkoxylation.

2. Process for making polymeric esterquats with asymmetric side chains, comprising the following steps:
(a) Esterification of a carboxylic acid blend, comprising (i) C₆-C₁₀ monocarboxylic acids, (ii) C₁₂-C₂₂ monocarboxylic acids, and (iii) dicarboxylic acids with alkanolamines in order to form a mixture of mono-, di- and trialkanolamine esters of said carboxylic acids, and subsequently
(b) Quaternisation of said ester mixture with alkylation agents, optionally after alkoxylation has taken place.

3. Process according to Claim 2, **characterised in that** said alkanolamines are used according to general formula **(I)**, 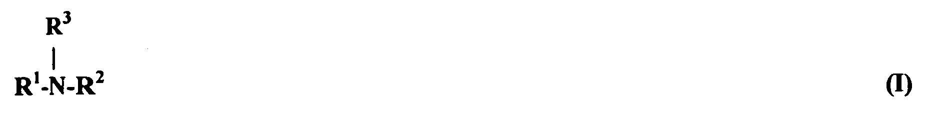 in which R¹ represents a hydroxyethyl radical, and R² and R³ independently of one another stand for hydrogen, methyl or a hydroxyethyl radical.

4. Process according to Claim 3, **characterised in that** triethanolamine is used.

5. Process according to any of Claims 2 to 4, **characterised in that** mixtures are used of
(i) Monocarboxylic acids selected from the group consisting of caproic acid, caprylic acid, 2-ethyl hexanoic acid, caprinic acid and their mixtures,
(ii) Monocarboxylic acids selected from the group consisting of lauric acid, myristic acid, palmitic acid, stearic acid, oleic acid, behenic acid and eruic acid and their mixtures, and
(iii) Dicarboxylic acids according to general formula **(II)**,
**HOOC-[X]-COOH** **(II)**
in which [X] stands for an optionally hydroxysubstituted alk(en)ylene group having 1 to 10 carbon atoms.

6. Process according to Claim 5, **characterised in that** adipic acid is used.

7. Process according to any of Claims 2 to 6, **characterised in that** carboxylic acid blends consisting of caprylic acid, stearic acid and adipic acid are used.

8. Process according to any of Claims 2 to 7, **characterised in that** mixtures of monocarboxylic acids of groups (i) and (ii) in molar ratios of 30:70 to 70:30 are used.

9. Process according to any of Claims 2 to 8, **characterised in that** monocarboxylic acids (i+ii) and dicarboxylic acids (iii) in molar ratios of 1:10 to 10:1 are used.

10. Process according to any of Claims 2 to 9, **characterised in that** alkylation agents selected from the group consisting of alkyl halides, aryl halides or dialkyl sulphates are used.

11. Process according to Claim 10, **characterised in that** methyl chloride, benzyl chloride or dimethyl sulphate are used.

12. Process according to any of Claims 2 to 11, **characterised in that** 95 to 105 Mol-% of the alkylation agent calculated on the molar amount of nitrogen in the ester mixture are used.

13. Use of polymeric esterquats according to Claim 1 for making cosmetic compositions.

14. Use according to Claim 13, **characterised in that** said cosmetic composition is a skin care composition.

15. Use according to Claim 13, **characterised in that** said cosmetic composition is a hair care composition.

16. Use according to Claims 13 and 15, **characterised in that** said hair care composition is a shampoo or a conditioner.

## Patentansprüche

1. Polymere Esterquats mit asymmetrischen Seitenketten, die durch Umsetzung von Alkanolaminen mit einer Mischung von:
(i) C₆-C₁₀-Monocarbonsäuren,
(ii) C₁₂-C₂₂-Monocarbonsäuren und
(iii) Dicarbonsäuren und
Quaternisierung der resultierenden Ester mit Alkylierungsmitteln, gegebenenfalls nach Alkoxylierung, erhältlich sind.

2. Verfahren zur Herstellung von polymeren Esterquats mit asymmetrischen Seitenketten mit den folgenden Schritten:
(a) Veresterung eines Carbonsäureblends, der (i) C₆-C₁₀-Monocarbonsäuren, (ii) C₁₂-C₂₂-Monocarbonsäuren und (iii) Dicarbonsäuren umfasst, mit Alkanolaminen zur Bildung einer Mischung von Mono-, Di- und Trialkanolaminestern der Carbonsäuren und anschließend
(b) Quaternisierung der Estermischung mit Alkylierungsmitteln, gegebenenfalls nach erfolgter Alkoxylierung.

3. Verfahren nach Anspruch 2, **dadurch gekennzeichnet, dass** man Alkanolamine der allgemeinen Formel (I), 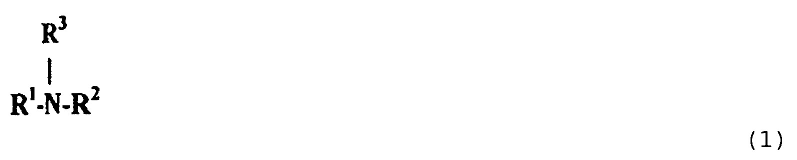 worin R¹ für einen Hydroxyethylrest steht und R² und R³ unabhängig voneinander für Wasserstoff, Methyl oder einen Hydroxyethylrest stehen, verwendet.

4. Verfahren nach Anspruch 3, **dadurch gekennzeichnet, dass** man Triethanolamin verwendet.

5. Verfahren nach einem der Ansprüche 2 bis 4, **dadurch gekennzeichnet, dass** man Mischungen von
(i) Monocarbonsäuren aus der Gruppe bestehend aus Capronsäure, Caprylsäure, 2-Ethylhexansäure, Caprinsäure und deren Mischungen,
(ii) Monocarbonsäuren aus der Gruppe bestehend aus Laurinsäure, Myristinsäure, Palmitinsäure, Stearinsäure, Ölsäure, Behensäure und Erucasäure und deren Mischungen und
(iii) Dicarbonsäuren der allgemeinen Formel (II),
**HOOC-[X]-COOH** **(II)**
worin [X] für eine gegebenenfalls hydroxysubstituierte Alk(en)ylengruppe mit 1 bis 10 Kohlenstoffatomen steht,
verwendet.

6. Verfahren nach Anspruch 5, **dadurch gekennzeichnet, dass** man Adipinsäure verwendet.

7. Verfahren nach einem der Ansprüche 2 bis 6, **dadurch gekennzeichnet, dass** man Carbonsäureblends aus Caprylsäure, Stearinsäure und Adipinsäure verwendet.

8. Verfahren nach einem der Ansprüche 2 bis 7, **dadurch gekennzeichnet, dass** man Mischungen von Monocarbonsäuren der Gruppen (i) und (ii) in Molverhältnissen von 30:70 bis 70:30 verwendet.

9. Verfahren nach einem der Ansprüche 2 bis 8, **dadurch gekennzeichnet, dass** man Monocarbonsäuren (i+ii) und Dicarbonsäuren (iii) in Molverhältnissen von 1:10 bis 10:1 verwendet.

10. Verfahren nach einem der Ansprüche 2 bis 9, **dadurch gekennzeichnet, dass** man Alkylierungsmittel aus der Gruppe bestehend aus Alkylhalogeniden, Arylhalogeniden oder Dialkylsulfaten verwendet.

11. Verfahren nach Anspruch 10, **dadurch gekennzeichnet, dass** man Methylchlorid, Benzylchlorid oder Dimethylsulfat verwendet.

12. Verfahren nach einem der Ansprüche 2 bis 11, **dadurch gekennzeichnet, dass** man 95 bis 105 Mol-% des Alkylierungsmittels, berechnet auf die molare Menge an Stickstoff in der Estermischung, verwendet.

13. Verwendung von polymeren Esterquats nach Anspruch 1 zur Herstellung von kosmetischen Zusammensetzungen.

14. Verwendung nach Anspruch 13, **dadurch gekennzeichnet, dass** es sich bei der kosmetischen Zusammensetzung um eine Hautpflegezusammensetzung handelt.

15. Verwendung nach Anspruch 13, **dadurch gekennzeichnet, dass** es sich bei der kosmetischen Zusammensetzung um eine Haarpflegezusammensetzung handelt.

16. Verwendung nach den Ansprüchen 13 und 15, **dadurch gekennzeichnet, dass** es sich bei der Haarpflegezusammensetzung um ein Shampoo oder einen Conditioner handelt.

## Revendications

1. Esterquats polymères à chaînes latérales asymétriques, pouvant être obtenus en faisant réagir des alcanolamines avec un mélange
(i) d'acides monocarboxyliques en C₆-C₁₀,
(ii) d'acides monocarboxyliques en C₁₂-C₂₂, et
(iii) d'acides dicarboxyliques, et
en quaternisant les esters résultants avec des agents d'alkylation, éventuellement après alcoxylation.

2. Procédé de préparation d'esterquats polymères à chaînes latérales asymétriques, comprenant les étapes suivantes :
(a) estérification d'un mélange d'acides carboxyliques comprenant (i) des acides monocarboxyliques en C₆-C₁₀, (ii) des acides monocarboxyliques en C₁₂-C₂₂, et (iii) des acides dicarboxyliques, avec des alcanolamines afin de former un mélange d'esters de mono-, di- et trialcanolamines desdits acides carboxyliques, puis
(b) quaternisation dudit mélange d'esters avec des agents d'alkylation, éventuellement après qu'une alcoxylation a eu lieu.

3. Procédé selon la revendication 2, **caractérisé en ce qu'**on utilise lesdites alcanolamines répondant à la formule générale (I) 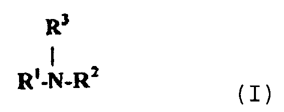 dans laquelle R¹ représente un radical hydroxyéthyle, et R² et R³, indépendamment l'un de l'autre, représentent un hydrogène ou un radical méthyle ou hydroxyéthyle.

4. Procédé selon la revendication 3, **caractérisé en ce qu'**on utilise la triéthanolamine.

5. Procédé selon l'une quelconque des revendications 2 à 4, **caractérisé en ce qu'**on utilise des mélanges
(i) d'acides monocarboxyliques choisis dans le groupe constitué par l'acide caproïque, l'acide caprylique, l'acide 2-éthylhexanoïque, l'acide caprinique et leurs mélanges,
(ii) d'acides monocarboxyliques choisis dans le groupe constitué par l'acide laurique, l'acide myristique, l'acide palmitique, l'acide stéarique, l'acide oléique, l'acide béhénique, l'acide érucique et leurs mélanges, et
(iii) d'acides dicarboxyliques de formule générale (II)
**HOOC-[X]-COOH** **(II)**
dans laquelle [X] représente un groupe alkylène ou alcénylène éventuellement hydroxysubstitué portant 1 à 10 atomes de carbone.

6. Procédé selon la revendication 5, **caractérisé en ce qu'**on utilise l'acide adipique.

7. Procédé selon l'une quelconque des revendications 2 à 6, **caractérisé en ce qu'**on utilise des mélanges d'acides carboxyliques constitués d'acide caprylique, d'acide stéarique et d'acide adipique.

8. Procédé selon l'une quelconque des revendications 2 à 7, **caractérisé en ce qu'**on utilise des mélanges d'acides monocarboxyliques des groupes (i) et (ii) dans des rapports molaires de 30:70 à 70:30.

9. Procédé selon l'une quelconque des revendications 2 à 8, **caractérisé en ce qu'**on utilise des acides monocarboxyliques (i+ii) et des acides dicarboxyliques (iii) dans des rapports molaires de 1:10 à 10:1.

10. Procédé selon l'une quelconque des revendications 2 à 9, **caractérisé en ce qu'**on utilise des agents d'alkylation choisis dans le groupe constitué par les halogénures d'alkyle, les halogénures d'aryle et les sulfates de dialkyle.

11. Procédé selon la revendication 10, **caractérisé en ce qu'**on utilise le chlorure de méthyle, le chlorure de benzyle ou le sulfate de diméthyle.

12. Procédé selon l'une quelconque des revendications 2 à 11, **caractérisé en ce qu'**on utilise 95 à 105 % en moles de l'agent d'alkylation, calculé par rapport à la quantité molaire d'azote dans le mélange d'esters.

13. Utilisation d'esterquats polymères selon la revendication 1 pour la préparation de compositions cosmétiques.

14. Utilisation selon la revendication 13, **caractérisée en ce que** ladite composition cosmétique est une composition de soin de la peau.

15. Utilisation selon la revendication 13, **caractérisée en ce que** ladite composition cosmétique est une composition de soin des cheveux.

16. Utilisation selon les revendications 13 et 15, **caractérisée en ce que** ladite composition de soin des cheveux est un shampooing ou un revitalisant capillaire.
